# EUROPEAN PATENT APPLICATION

(11) **EP 4 732 673 A1**
(43) Date of publication of application: **29.04.2026**
(21) Application number: 24216588.4
(22) Date of filing: 29.11.2024
(51) Int. Cl.: A23J 1/00, A23J 3/20, C07K 14/405, C07K 1/14, C07K 1/34, C07K 1/36, C11B 1/04, C11B 1/06, C11B 1/10, C11B 3/00, A61K 31/722, A61K 36/02, C12N 1/12, C12N 1/06, B01D 21/26

(54) **CASCADING FRACTIONAL COMPONENT EXTRACTION FROM MICROALGAE**

(30) Priority: 25.10.2024 ES 202431975 U
(71) Applicant: Leitat (Acondicionamiento Tarrasense), 08788 Vilanova del Camí Barcelona (ES); Latil de Ros, Derek Georges José, 08500 Vic Barcelona (ES)
(72) Inventor: Latil de Ros, Derek Georges José, 08500 Vic (Barcelona) (ES); Fabregat Torrents, Cristina, 08711 ODena (ES); Jorba Rafart, Montserrat, 08700 Igualada (ES); Ortiz Seco, Laura, 08720 Vilafranca del Penedés (ES)
(74) Representative: Bringer IP

(57) **Abstract**

The cascaded extraction of fractional components of an algal biomass includes an initial disruption of an algal cell mass and the centrifugation of the disrupted algal cell mass into each of an oil fraction, an aqueous fraction and a solid fraction. Thereafter, the oil fraction may be purified into an algal oil, while the aqueous fraction may be micro-filtrated and dried into a protein, and the solid fraction may be enzymatically digested, macerated, precipitated and dried into a chitosan.

## Description

### BACKGROUND OF THE INVENTION

### Field of the Invention

The present invention relates to the field of microalgae processing and more particularly to the extraction of fractional components from microalgae.

### Description of the Related Art

Algae have been part of the human diet for thousands of years. In the form of a macroalgae, recent data suggests that the global harvest of seaweed has become significant. In addition to macroalgae, some microalgae have been cultivated for foods and food additives. To wit, while an enormous quantity of seaweed has been consumed by humans in the form of kelp or nori/laver, an ever-increasing quantity of microalgae also has been consumed in recent years both as a protein source in the form of spirulina and chlorella, and also as a thickening agent in the form of agars, alginates and carrageenans. While the human consumption of algal foods varies by location, overall, the trend towards increasing nutritional demand for algal products on a global basis stems from a greater focus on health and wider use of food additives.

Microalgae offer substantial environmental benefits compared with traditional protein sources. Microalgae require less than 2.5 square meters of land per kilogram of protein produced, whereas beef uses nearly one-hundred fifty thousand square meters per kilogram protein. Microalgae can also be cultivated on nonarable land and use minimal amounts of fresh water. Unfortunately, microalgae processing technology is highly underdeveloped, making microalgae expensive, particularly regarding harvesting and dewatering and fractionalization.

Indeed, conventional mechanisms for the fractionation of microalgae focus upon obtaining merely one ingredient for one industrial application at a time. Thus, the conventional fractionalization of microalgae comes at a high cost, producing low yields. Significant research and technological improvements are necessary before microalgae can become a widely utilized ingredient source. An integrated approach enabling the extraction of, not only proteins, but also other valuable components, such as oils and polysaccharides, is required to maximize the overall value derived from microalgae.

### BRIEF SUMMARY OF THE INVENTION

Embodiments of the present invention address deficiencies of the art in respect to the production of microalgae and provide a novel and non-obvious process for the cascaded extraction of fractional components of an algal biomass. In an embodiment of the invention, a method for the cascading extraction of fractional components of microalgae includes an initial disruption of an algal cell mass and the centrifugation of the disrupted algal cell mass into each of an oil fraction, an aqueous fraction and a solid fraction. Thereafter, the oil fraction may be purified into an algal oil, while the aqueous fraction may be micro-filtrated and dried into a protein, and the solid fraction may be enzymatically digested, macerated, precipitated and dried into chitosan.

Different aspects of the embodiment are specified herein including the following variations which may occur alone or in combination with one another:
- The disruption of the algal mass is a high pressure homogenization (HPH) of the algal mass subsequent to a centrifugation of the algal mass at room temperature followed by a dissolution of the centrifugated algal mass in a basic buffer at room temperature.
- The centrifugation of the disrupted algal cell mass is a three-phase centrifugation from 2,000 relative centrifugal force to 10,000 relative centrifugal force.
- The micro-filtration includes a filtration of a membrane or two or more membranes in sequence of one to one-thousand kilodaltons, and as one example, with a sequence of membranes of respective pore sizes of four kilodaltons, ten kilodaltons and twenty kilodaltons.
- The maceration is a dual basic maceration for twelve to seventy two hours, followed by an acid maceration for twelve to seventy hours.
- The maceration is followed by a centrifugation of the solid fraction at 2,000 relative centrifugal force to 10,000 relative centrifugal force for between five and twenty minutes.

In another embodiment of the invention, a method for the cascading extraction of fractional components of microalgae includes disrupting an algal cell mass by submitting the algal cell mass to high-pressure homogenization in order to produce a disrupted microalgal biomass and centrifugating the disrupted microalgal biomass into each of an oil fraction, an aqueous fraction and a solid fraction. Thereafter, the oil fraction may be purified into an algal oil, the aqueous fraction may be micro-filtrated and dried into a protein, and and the solid fraction may be enzymatically digested, basic macerated, and then separated into a liquid peptide rich fraction and a remaining solid fraction pellet. The peptide fraction may be dried. As such, the pellet can be acidically macerated to produce an additional aqueous fraction and a resulting solid fraction. The additional aqueous fraction is then precipitated and washed in order to produce a final solid chitosan rich fraction..

In this way, by extracting multiple different valuable fractional components during the processing of the algal biomass, the economics of the production of microalgae as a protein source can be dramatically improved so as to result in a cost effective substitute protein source from traditional livestock.

Additional aspects of the invention will be set forth in part in the description which follows, and in part will be obvious from the description, or may be learned by practice of the invention. The aspects of the invention will be realized and attained by means of the elements and combinations particularly pointed out in the appended claims. It is to be understood that both the foregoing general description and the following detailed description are exemplary and explanatory only and are not restrictive of the invention, as claimed.

### BRIEF DESCRIPTION OF THE SEVERAL VIEWS OF THE DRAWINGS

The accompanying drawings, which are incorporated in and constitute part of this specification, illustrate embodiments of the invention and together with the description, serve to explain the principles of the invention. The embodiments illustrated herein are presently preferred, it being understood, however, that the invention is not limited to the precise arrangements and instrumentalities shown, wherein:
Figure 1 is pictorial illustration of a process for cascading extraction of fractional components of microalgae;
Figure 2 is a flow chart illustrating a process for the cascading extraction of fractional components of microalgae; and,
Figure 3 is a block diagram illustrating an apparatus adapted for the cascading extraction of fractional components of microalgae.

### DETAILED DESCRIPTION OF THE INVENTION

Embodiments of the invention provide for the cascading extraction of fractional components of microalgae. In accordance with an embodiment of the invention, an algal mass may be disrupted and separated by centrifugation into each of an oil fraction, an aqueous fraction and a solid fraction. From the oil fraction, microalgae oil can be extracted, while from the aqueous fraction, subject to microfiltration and drying, proteins may be extracted. Finally, from the solid fraction, produced by the enzymatic digestion, and followed thereafter first by basic and subsequently acidic maceration and then precipitation and drying, chitosans may be extracted. Concurrently, the aqueous portion of the solid fraction macerated under basic conditions, may be fed to the dryer of the aqueous fraction. In this way, the process of protein production from the aqueous fraction of the microalgae can be optimized through the harvesting of the other fractions of the microalgae.

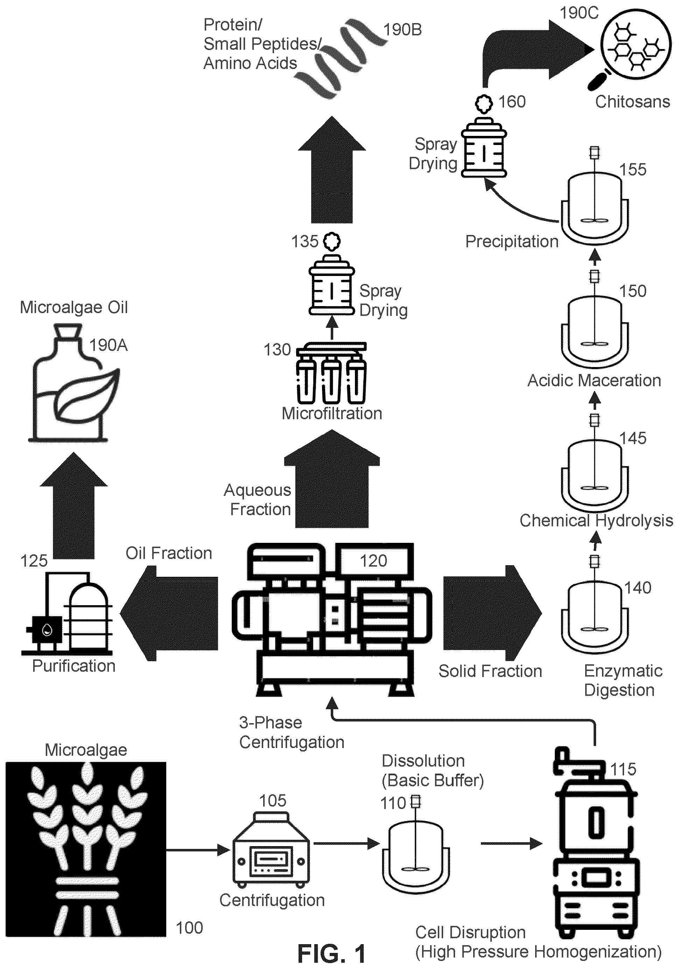

In further illustration, Figure 1 pictorially shows a process for cascading extraction of fractional components of microalgae. As shown in Figure 1, microalgae 100 can be subjected to centrifugation 100 followed by dissolution 110 in a basic buffer. Cell disruption 115 is then performed through high-pressure homogenization with the resulting slurry subjected to three-phase centrifugation 120. The three-phase centrifugation 120 produces three separate fractions: an oil fraction, an aqueous fraction and a solid fraction. As to the oil fraction, the oil fraction can be subjected to purification 125 in order to produce microalgae oil 190A.

As to the aqueous fraction, the aqueous fraction can be subjected first to microfiltration 130 and thereafter, drying 135 in order to separate proteins and pigments from small colourless peptides and amino acids 190B. Of note, the aqueous fraction can be subjected to enzymatic hydrolysis, without which a permeate protein fraction formed by small proteins is produced along with peptides and free amino acids of no more than 5%. The enzymatic hydrolysis step thus breaks the proteins so as to produce peptides and free amino acids in both a concentrate fraction and also a permeate fraction. Thereafter, microfiltration 130 is performed and then drying 135 in order to increase the colorless peptides and amino acid fraction.

Finally, as to the solid fraction, the solid fraction can be subjected to enzymatic digestion 140, the solid output from the enzymatic digestion 140 is provided to chemical hydrolysis 145 and acidic maceration 150. The resulting liquid from the acidic maceration 150 is subjected to precipitation 155 and the solid output is then subjected to drying 160 so as to produce chitosans 190C.

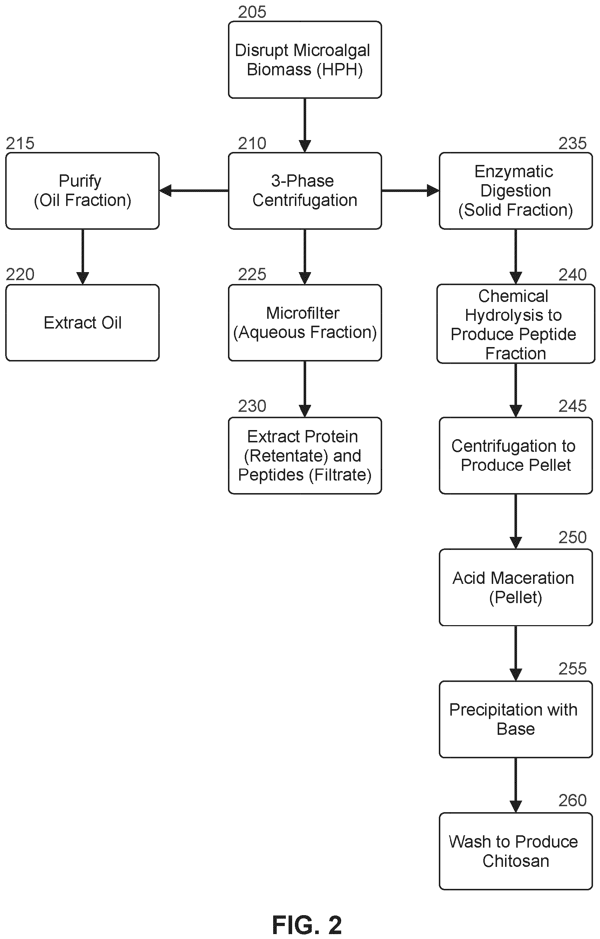

In further illustration, Figure 2 is a flow chart illustrating a process for the cascading extraction of fractional components of microalgae. Beginning in block 205, a microalgal biomass is disrupted by way of centrifugation to ten to twenty percent dry matter at room temperature, dissolved in a basic buffer of pH eight (8) through twelve (12) at room temperature, and then high pressure homogenized at one-thousand five thousand (500) to two thousand five hundred (2,500) bar from once to upwards of six cycles at less than fifty degrees Celsius.

In block 210, the resulting cell disrupted micro algal mass is three-phase centrifuged either in a continuous or variable centrifuge process, in the latter instance from two thousand (2,000) relative centrifugal force to ten thousand (10,000) relative centrifugal force. The result is each of an oil fraction, an aqueous fraction and a solid fraction. As to the oil fraction, in block 215 the oil fraction is purified to produce an extract in block 220 of oil. As to the aqueous fraction, in block 225 the aqueous fraction is microfiltered with one or more membranes in sequence of one to one-thousand kilodaltons, and as one example, with at least three membranes of four, ten and twenty kilodaltons. The result is then dried so as to produce an extract in block 230 of protein as the retentate and peptides as the filtrate.

As to the solid fraction, in block 235 the solid fraction is subjected to enzymatic digestion in a mixing tank with the solid output experiencing chemical hydrolysis in block 240. Then, the solid result is the centrifuged in block 245 either continuously or from two thousand (2,000) relative centrifugal force to ten thousand (10,000) relative centrifugal force, producing as a result a peptide fraction and pellets.

In block 250, the pellets are then acidically macerated in a solution of pH two (2) to pH six (6) with the resulting liquid precipitating in block 255 according to a base solution of sodium hydroxide and sodium bicarbonate. Finally, in block 260 the solid result from the precipitation is washed with water and then centrifuged from two thousand (2,000) relative centrifugal force to ten thousand (10,000) relative centrifugal force for between five and twenty minutes at less than fifty degrees Celsius to produce a chitosan.

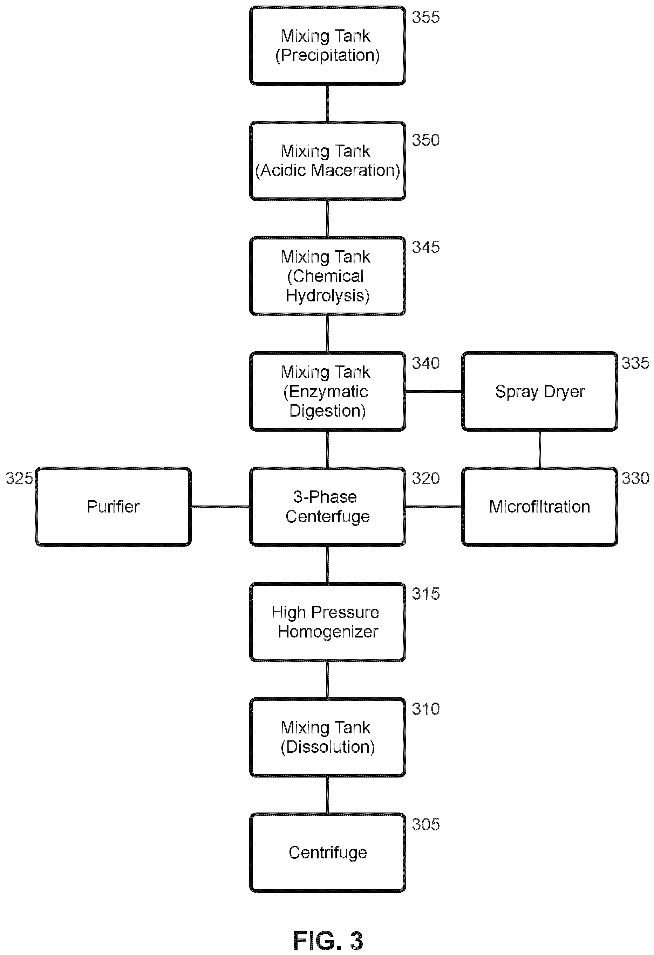

The process described in connection with Figures 1 and 2 can be performed within an apparatus adapted for the cascaded extraction of the fractional components of microalgae. In more particular illustration, Figure 3 is a block diagram illustrating an apparatus adapted for the cascading extraction of fractional components of microalgae. As shown in Figure 3, each of a centrifuge 305, a mixing tank for dissolution 310 and a high pressure homogenizer 315 can be positioned to engage in the cellular disruption of a microalgal/chlorella biomass in a base of sodium hydroxide. A three-phase centrifuge 320 is then provided which can process the cellularly disrupted slurry into each of an oil fraction, an aqueous fraction and a solid fraction.

A purifier 325 processes the oil fraction in order to purify and stabilize algal oils. Microfiltration 330, which can include a sequence of one or more members ranging from one to one-thousand kilodaltons, and as a particular example a sequence of membranes of 4, 10 and 20 kilodaltons, processes the aqueous fraction which, in concert with a dryer 335, produce both small peptides and amino acids as the filtrate of the microfiltration 330, and also proteins and pigments as the retentate of the microfiltration 330.

Finally, a series of mixing tanks 340, 345, 350, 355 process in sequence the solid fraction produced by the three-phase centrifuge 320. In particular, a first mixing tank 340 performs enzymatic digestion of the solid fraction with proteases. The second mixing tank 345 performs chemical hydrolysis of the output from the first mixing tank 340. The third mixing tank 350 performs acidic maceration of the output of the second mixing tank 345 in a pH 2 to pH 6 solution for twelve to seventy-two hours. Finally, a fourth mixing tank 355 precipitates the liquid output of the third mixing tank 350 in a solution of pH 7 to pH 11 which when followed by washing and centrifugation, produces a chitosan.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "include", "includes", and/or "including," when used in this specification, specify the presence of stated features, integers, steps, operations, elements, and/or components, but do not preclude the presence or addition of one or more other features, integers, steps, operations, elements, components, and/or groups thereof.

The corresponding structures, materials, acts, and equivalents of all means or step plus function elements in the claims below are intended to include any structure, material, or act for performing the function in combination with other claimed elements as specifically claimed. The description of the present invention has been presented for purposes of illustration and description but is not intended to be exhaustive or limited to the invention in the form disclosed. Many modifications and variations will be apparent to those of ordinary skill in the art without departing from the scope and spirit of the invention. The embodiment was chosen and described in order to best explain the principles of the invention and the practical application, and to enable others of ordinary skill in the art to understand the invention for various embodiments with various modifications as are suited to the particular use contemplated.

Having thus described the invention of the present application in detail and by reference to embodiments thereof, it will be apparent that modifications and variations are possible without departing from the scope of the invention defined in the appended claims as follows:

## Claims

1. A method for the cascading extraction of fractional components of microalgae, the method comprising:
disrupting an algal cell mass;
centrifugating the disrupted algal cell mass into each of an oil fraction, an aqueous fraction and a solid fraction;
purifying the oil fraction into an algal oil;
micro-filtrating and drying the aqueous fraction into a protein; and,
enzymatically digesting, macerating, precipitating and then drying the solid fraction into a chitosan.

2. The method of claim 1, wherein the disruption of the algal mass is a high pressure homogenization of the algal mass subsequent to a centrifugation of the algal mass at room temperature followed by a dissolution of the centrifugated algal mass in a basic buffer at room temperature.

3. The method of claim 1, wherein the centrifugation of the disrupted algal cell mass is a three-phase centrifugation from 2,000 relative centrifugal force to 10,000 relative centrifugal force.

4. The method of claim 1, wherein the micro-filtration includes a filtration of one or more membranes in sequence of one to one-thousand kilodaltons, and as one example, with a three part filtration through a sequence of membranes of respective pore sizes of four kilodaltons, ten kilodaltons and twenty kilodaltons.

5. The method of claim 1, wherein the maceration is a dual basic maceration for twelve to seventy two hours followed by an acid maceration for twelve to seventy two hours.

6. The method of claim 1, wherein the maceration is followed by a centrifugation of the solid fraction at 2,000 relative centrifugal force to 10,000 relative centrifugal force.

7. A method for the cascading extraction of fractional components of microalgae, the method comprising:
disrupting an algal cell mass by submitting the algal cell mass to high-pressure homogenization in order to produce a disrupted microalgal biomass;
centrifugating the disrupted microalgal biomass into each of an oil fraction, an aqueous fraction and a solid fraction;
purifying the oil fraction into an algal oil, micro-filtrating and drying the aqueous fraction into a protein, and enzymatically digesting, basic macerating, precipitating and then drying the solid fraction into a peptide fraction and a remaining solid fraction pellet; and,
acidic macerating the pellet to produce an additional aqueous fraction and a resulting solid fraction, precipitating the additional aqueous fraction to produce a final solid fraction and washing and centrifuging the final solid fraction to product a chitosan.
